# EUROPÄISCHE PATENTANMELDUNG

(11) **EP 1 159 973 A1**
(43) Veröffentlichungstag der Anmeldung: **05.12.2001**
(21) Anmeldenummer: 01810476.0
(22) Anmeldetag: 15.05.2001
(51) Int. Cl.: A61L 27/30, A61L 31/08, A61L 33/02

(54) **Antihaftbeschichtung sowie deren Verwendung**

(30) Priorität: 29.05.2000 CH 20001078
(71) Anmelder: Lerf, Reto, Dr., 5037 Muhen (CH)
(72) Erfinder: Lerf, Reto, Dr., 5037 Muhen (CH)
(74) Vertreter: Schwander, Kuno

(57) **Zusammenfassung**

Die Erfindung betrifft eine Antihaftbeschichtung für insbesondere metallische Werkstoffe. Bevorzugtes Anwendungsgebiet der Erfindung ist die Medizinaltechnik. So eignet sich die erfindungsgemässe Beschichtung vor allem als Implantat- und Prothesenoberfläche oder als Oberfläche für Implantate zur operativen Knochenbruchheilung. Die erfindungsgemässe Beschichtung besteht im wesentlichen aus einer titanhaltigen Legierung mit einer quasikristallinen Phase, deren Anteil mindestens 50 Vol.% beträgt. Die erfindungsgemässe Beschichtung ist insbesondere sehr hart und gegenüber vielen Flüssigkeiten korrosionsbeständig. Sie besitzt zudem ausgezeichnete Antihafteigenschaften und zeigt ein sehr gutes Verschleiss- und Reibverhalten.

## Beschreibung

Die Erfindung betrifft eine Antihaftbeschichtung für Festkörper, insbesondere metallische Werkstoffe.

Bevorzugtes Anwendungsgebiet der Erfindung ist die Medizinaltechnik. So eignet sich die erfindungsgemässe Beschichtung vor allem als Implantat- und Prothesenoberfläche, zum Beispiel als Oberfläche mit Blutkontakt bei GefässImplantaten, als Gleitfläche für Gelenkprothesen, oder als Oberfläche für Implantate zur operativen Knochenbruchheilung, also als Beschichtung von Knochenplatten, Knochenschrauben, Marknägel, Klammern etc.

Bekannte Implantate und Prothesen bestehen aus unterschiedlichen Werkstoffen - nachfolgend auch Substrate genannt - wie nichtrostendem Stahl, Titan, Keramik, Kunststoff oder dergleichen. Sie werden gewöhnlich in situ angepasst und anschliessend für einen verhältnismässig langen Zeitraum und zum Beispiel als Körperteil-Ersatz in einen tierischen oder menschlichen Körper eingesetzt.

Mit dem Ziel, eine durch den chemischen Angriff der Körperflüssigkeit hervorgerufene Korrosion sowie auch einen erheblichen Materialverschleiss durch Abrieb zu vermeiden, werden heute Implantate und Prothesen mit einer speziellen Oberflächenbeschichtung überzogen, wozu sich als Komponenten der Beschichtungsmaterialien in erster Linie Hartstoffe wie zum Beispiel Titannitrid, Zirkonnitrid, Titankarbid und Borkarbid eignen.

Die Kenntnisse über die Nachteile der so behandelten Substrate sind ebenso verbreitet wie die Überzeugung, dass diese Art der Beschichtung nötig ist, um eine hohe Abriebfestigkeit sowie eine hohe Korrosionsbeständigkeit zu erhalten. Ein grosser Nachteil besteht im wesentlichen darin, dass die bekannten Oberflächenbeschichtungen eine Neigung zum Abplatzen oder Abspringen haben und mit der Zeit beginnen, sich vom Substrat zu lösen, so dass dadurch die Abriebfestigkeit und Korrosionsbeständigkeit der Implantate und Prothesen verringert werden.

Bei kardiovaskulären Implantaten und Medizingeräten, also z.B. künstlichen Herzklappen oder Herz-Lungenmaschninen, ist bekannt, dass die Blutkompatibilität der mit dem Blut in Kontakt stehenden Oberflächen die entscheidende Rolle für den Erfolg eines solchen Implantates oder Gerätes ist. Bei Kunststoffen wurden mit viel Aufwand Oberflächenmodifikationen eingeführt, die eine Verbesserung der Blutkompatibilität erzielen. Für Metalle im Kontakt mit dem Blutkreislauf fehlen bisher überzeugende Lösungen zur Reduktion der Probleme im Zusammenhang mit der Blutkompatibilität. Namentlich die Haftfestigkeit von polymeren Beschichtungen ist unbefriedigend.

Neben den Implantaten und Prothesen der vorgenannten Art, deren Oberflächenbeschichtungen gewöhnlich biokompatibel sind, gibt es auch Implantate, die nur für eine bestimmte Zeit in den Körper eingesetzt und nach einer üblicherweise mehrere Wochen andauernden Behandlungs- und Regenerationsphase vom Chirurg wieder entfernt werden. Zu diesen Implantaten gehören die Knochenplatten und Knochenschrauben. Sie bestehen vorzugsweise aus Titan oder einer Legierung auf Titanbasis. Unter einer Legierung auf Titanbasis versteht man in diesem Zusammenhang eine Legierung, die entweder einen Titangehalt von mehr als 30 Gew.% aufweist oder bei der wenigstens das Legierungselement mit dem grössten Prozentsatz Titan ist.

Bei den modernen Methoden der operativen Behandlung von Knochenbrüchen werden die Knochenfragmente durch die Platten und Schrauben unter verhältnismässig hohen Druck gebracht. Es treten also beim Einsetzen der Schrauben in die Platte und beim Belasten der operierten Knochen grosse Kräfte auf, welche eine hohe Reibung zwischen den Teilen verursachen. Es sind also insbesondere an die Oberflächenbeschaffenheit solcher Implantate hohe Anforderungen bezüglich Verschleissfestigkeit und Korrosionsbeständigkeit gestellt. Dazu kommt, dass die nach einer bestimmten Zeit wieder zu entfernenden Implantate so beschaffen sein müssen, dass das umliegende Knochengewebe am Implantat nicht anwächst.

Es ist auch bekannt, die Gleitflächen von Gelenkprothesen mit Schutzschichten zu versehen, um ihre Verschleissfestigkeit und Korrosionsbeständigkeit zu erhöhen. In der Praxis hat sich gezeigt, dass die für derartige Schichten bisher verwendeten Werkstoffe bezüglich ihrer Gleiteigenschaften, ihrer Haftung auf dem Substrat und bezüglich ihrer Antihafteigenschaft gegenüber tierischem oder menschlichem Gewebe nicht allen Anforderungen genügen.

Ferner werden sowohl bei medizinischen als auch analytischen Geräten häufig apparative Teile eingesetzt, die gegenüber Körperflüssigkeiten nur begrenzt beständig oder zu solchen kompatibel sind und zum Anhaften von Proteinen aus proteinhaltigen Lösungen neigen. Anhaftende proteinhaltige Verunreinigungen führen in diesen Fällen sehr oft zu einer Messungenauigkeit, was dann eine Nacharbeit der Messungen zur Folge hat. In diesen Fällen müssen solche Geräte mit verhältnismässig grossem Aufwand regelmässig gewartet und gereinigt werden.

In Kenntnis des bekannten Standes der Technik liegt der Erfindung die Aufgabe zu Grunde, die erkannten Nachteile zu beheben und eine alternative Schutzschicht für Festkörper, insbesondere metallische Festkörper, nachfolgend auch Substrate genannt, zur Verfügung zu stellen, die eine verhältnismässig hohe Verschleissfestigkeit und gute Reibungseigenschaften besitzt sowie möglichst vielfältig einsetzbar ist. Dabei soll insbesondere eine verbesserte Beschichtung für Prothesengleitflächen und Implantate geschaffen werden, die eine verhältnismässig hohe Korrosionsbeständigkeit und gute Antihafteigenschaften gegenüber Proteinen und tierischem oder menschlichem Gewebe besitzen.

Erfindungsgemäss wurde erkannt, dass diese Aufgabe durch eine Antihaftbeschichtung mit den Merkmalen des Anspruchs 1 gelöst wird.

Vorteilhafte Ausführungsformen der Erfindung gehen aus den abhängigen Ansprüchen hervor.

Die Erfindung betrifft ferner die Verwendung der Antihaftbeschichtung gemäss den Ansprüchen 7 und 13 - 17 sowie auch Implantate und Prothesen gemäss den Ansprüchen 8 bis 12.

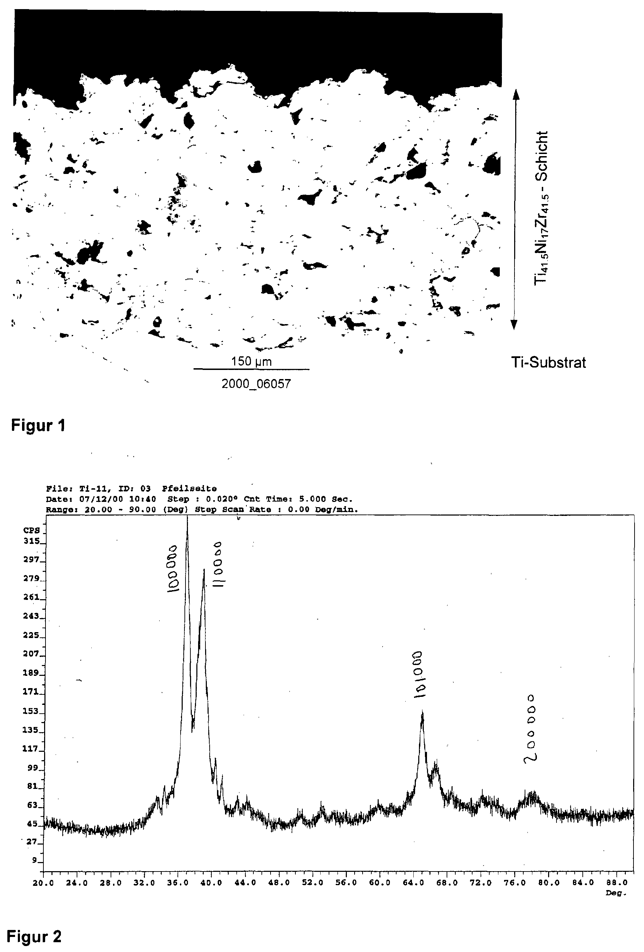

Zur nachfolgend im Detail beschriebenen Erfindung zeigen die Figur 1 einen Querschliff durch eine erfindungsgemässe Beschichtung und die Figur 2 ein Röntgen-Beugungsdiagramm der Beschichtung gemäss Figur 1.

Die erfindungsgemässe Beschichtung besteht im wesentlichen aus einer titanhaltigen Legierung mit einer mittels Röntgenbeugung gemessenen quasikristallinen Phase, deren Anteil mindestens 50 Vol.% beträgt. Unter quasikristalliner Phase versteht man hierbei einen neuen Zustand des Festkörpers, der sich von den beiden bekannten Zuständen kristallin und amorph insofern unterscheidet, dass eine translatorische Ordnung des (Quasi-)Kristallgitters auf lange Distanz kombiniert ist mit einer in der klassischen Kristallographie verbotenen 5-fachen (ikosaedrisch), 8-fachen (oktogonal), 10-fachen (dekagonal) oder 12-fachen (dodekagonal) Symmetrieachse.

Die erfindungsgemässen und nachfolgend auch als Legierungen bezeichneten Beschichtungen sind sehr hart und gegenüber vielen Flüssigkeiten korrosionsbeständig. Sie besitzen des weiteren die geforderten Antihafteigenschaften und zeigen ein sehr gutes Verschleiss- und Reibverhalten. Sehr gute Eigenschaften ergeben sich insbesondere dann, wenn die Dicke der auf das Substrat applizierten Beschichtung zwischen 0.1 µm und 500 µm liegt. So werden zum Beispiel bei Schichten, die mittels PVD-Verfahren aufgetragen werden, Dicken von 1 µm bis 4 µm bevorzugt. Mit Plasmabeschichten werden vorzugsweise Schichten von 50 µm bis 150 µm aufgetragen.

Eine besonders zweckmässige Beschichtung der erfindungsgemässen Art ist dadurch gekennzeichnet, dass sie folgende, im stöchiometrischen Zahlenverhältnis der Atome angegebene, Legierungsbestandteile aufweist:

| | |
|---|---|
| 35 - 65 at.% | Ti |
| 8 - 50 at.% | Zr |
| 10 - 30 at.% | eines oder mehrerer der Elemente Ni, Co, Fe, Mn, Pd, Ag |

Konkrete Beispiele für Legierungen dieser ersten Kombinationsauswahl sind: Ti₆₅Fe₂₅Zr₁₀; Ti₆₀Fe₂₅Zr₁₅; Ti₅₃Co₂₀Zr₂₇; Ti₆₅Ni₂₅Zr₁₀; Ti₆₀Ni₂₅Zr₁₅; Ti₅₃Ni₂₀Zr₂₇; Ti₅₀Ni₁₉Zr₃₁; Ti₄₅Ni₁₇Zr₃₈, Ti₄₄Ni₁₆Zr₄₀ und Ti_{41.5}Ni₁₇Zr_{41.5}.

Bei einer anderen zweckmässigen Ausführungsform der Erfindung sind die Hauptbestandteile der Legierung in folgenden atomaren Verhältnissen vorhanden:

| | |
|---|---|
| 40 - 75 at.% | Ti |
| 3 - 25 at.% | Si |
| 15 - 50 at.% | eines oder mehrerer der Elemente Cr, Mn, V, Fe, Nb, Mo, Ta |
| 0 - 17 at.% | O₂ zur Stabilisierung der quasikristallinen Struktur |

Konkrete Beispiele für Legierungen dieser zweiten Kombinationsauswahl sind: Ti₆₈Fe₂₆Si₆; Ti₆₀Mn₃₇Si₃ und Ti₆₄Cr₃₂Si₄, oder Ti₆₈₋ₓCr₃₂Siₓ, wobei gilt 6 < x < 18, zum Beispiel Ti₆₀Cr₃₂Si₈; Ti₇₃₋ₓV₂₇Siₓ, wobei gilt 13 < x < 25, zum Beispiel Ti₅₇V₂₇Si₁₆; und Ti₇₅₋ₓCr₂₅Siₓ, wobei gilt 10 < x < 20, zum Beispiel Ti₆₅Cr₂₅Si₁₀.

Legierungen der erfindungsgemässen Art haben auch eine gute Haftfestigkeit auf dem Substrat, welches vorzugsweise aus einem Metall besteht, in dem Eisen, Kobalt oder Titan zumindest als Basismaterial enthalten ist. Selbstverständlich kann das Substrat auch ein in der Implantat-Technik gebräuchlicher anderer Werkstoff sein, so zum Beispiel ein Kunststoff, ein Reinmetall oder ein Keramikwerkstoff.

Im Rahmen der Erfindung können die erfindungsgemässen Beschichtungen auch aus zwei oder mehreren Schichten verschiedener Zusammensetzung hergestellt sein. In diesem Fall ist aber mindestens die äusserste Schicht als quasikristalline Legierung der erfindungsgemässen Art ausgebildet.

Zum Aufbringen der quasikristallinen Schicht auf ein Substrat können die verschiedensten, dem Fachmann bekannte Verfahren eingesetzt werden. So kann die Beschichtung eines Substrates mittels dem bekannten Plasma-Spritzverfahren erfolgen. Bei dieser Technik wird ein elektrischer Lichtbogen zum Erhitzen verschiedener Gase, wie Luft, Stickstoff oder Wasserstoff, benutzt. Die Gase werden dabei aus einem Ring mit hoher Geschwindigkeit ausgestossen und erzeugen eine charakteristische Flamme. Das vorzugsweise pulverförmige Beschichtungsmaterial wird dann in die Flamme eingeführt und die geschmolzenen Teilchen auf das zu überziehende Substrat aufgesprüht. Diesem Verfahren verwandt ist das Flammspritzen und das Hochgeschwindigkeitsflammspritzen.

Eine andere verwendbare Technik zum Abscheiden der quasikristallinen Legierung auf das Substrat ist das physikalische Bedampfen, auch PVD genannt (Physical-Vapor-Deposition). Bei diesem Verfahren wird ein Barren eines auf dem Substrat abzuscheidenden Materials in einer evakuierten Kammer angeordnet. Das obere Ende des Barren wird dann durch eine intensive Wärmequelle (z. B. Elektronenstrahl oder Laser) erhitzt, so dass es schmilzt und ein Schmelzbad bildet. Ein Teil des sehr heissen, geschmolzenen Materials verdampft und kondensiert anschliessend auf dem Substrat, so dass dadurch graduell ein Überzug aufgebaut wird.

Die erfindungsgemässen Beschichtungen lassen sich mittels des Plasma-Sprühverfahrens, mittels des PVD-Verfahrens oder mittels des ebenfalls bekannten CVD-Verfahrens (Chemical Vapor Deposition) verhältnismässig leicht auf das Substrat abscheiden. Diese Verfahren erlauben insbesondere auch ein problemloses Abscheiden des Überzugmaterials auf gekrümmte Oberflächen mit komplizierter Oberflächengeometrie.

Das Auftragen der erfindungsgemässen Legierung auf ein Substrat ist aber auch mittels anderen thermischen Verfahren, so etwa mittels bekannten Schweiss- und Lötverfahren, oder durch das dem Fachmann ebenfalls bekannte Laserbeschichtungsverfahren, oder durch eine Kombination von Verfahren der genannten Art möglich.

Die Figuren 1 und 2 zeigen charakteristische Merkmale einer erfindungsgemässen Beschichtung. So stellt die Figur 1 einen Querschliff durch die quasikristalline Schicht Ti_{41.5}Ni₁₇Zr_{41.5} auf Titansubstrat dar. Diese Schicht mit einer Dicke von ca. 0,3 mm wurde mittels Vakuum-Plasmaspritzen aufgebracht. Figur 2 seinerseits zeigt ein Röntgen-Beugungsdiagramm der quasikristallinen Schicht Ti_{41.5}Ni₁₇Zr_{41.5} aus Figur 1. Die Beugungsmaxima können eindeutig als ikosaedrische Phase mit Quasigitterkonstanten 5,18 A indexiert werden

Ein mit der erfindungsgemässen Legierung beschichtetes und für die Knochenheilung vorgesehenes Implantat weist gegenüber den derzeit gebräuchlichen Implantaten derselben Art mehrere Vorteile auf:
So besitzt es eine grössere Abriebfestigkeit als ein Implantat ohne Oberflächenschicht, was besonders für jene Implantate aus Titan oder Titan-Legierung wichtig ist, die sich an einem anderen, ebenfalls aus diesem Metall bestehenden Implantat, reiben, wie das zum Beispiel beim Einschrauben von Schrauben in eine Kompressionsplatte der Fall ist.
Ein solches Implantat besitzt ferner auch eine grössere Korrosionsbeständigkeit gegenüber Gewebeflüssigkeiten, und zwar nicht nur auf den grossflächigen Teilen seiner Oberfläche, sondern, was besonders wichtig ist, auch in den für Korrosionsangriffe besonders empfindlichen, verhältnismässig kleinen Vertiefungen und Öffnungen, wie Schraubenlöchern und Schraubenschlitzen.
Schliesslich zeigt das Implantat auch die vorstehend genannte Antihafteigenschaft gegenüber menschlichem und tierischem Gewebe. Dies hat den bedeutenden Vorteil, dass eine nachträgliche Entfernung des Implantates ohne nennenswerte Schwierigkeiten möglich ist.

Neben der Beschichtung von Implantaten, wie Knochenplatten, Knochenschrauben und dergleichen, ist eine andere bevorzugte Anwendung der Erfindung bei der Herstellung von Hüftgelenk-Kniegelenk und Schultergelenkprothesen zu sehen. So ist es für den konkreten Fall des Kniegelenkes von Vorteil, die Gelenkfläche der Kondyle mit einer 50 bis 150 µm dicken quasikristallinen Schicht der erfindungsgemässen Art zu versehen, und diese Oberfläche auf bekannte Art und Weise Hochglanz zu polieren. Das Tibiaplateau kann nach dem Stand der Technik aus Polyethylen mit ultrahohem Molekulargewicht (UHMWPE) gefertigt werden. Beide Teile bilden eine Prothese mit für die Gelenkfunktion optimalen Gleiteigenschaften und minimem Verschleiss.

Abschliessend sei noch darauf hingewiesen, dass die beiden vorstehend beschriebenen Beispiele für den Einsatz der erfindungsgemässen Legierung nur eine Auswahl von mehreren möglichen Anwendungsformen der Erfindung darstellen und dass diese universell einsetzbar ist. So lassen sich auch Gefässimplantate mit der erfindungsgemässen Legierung beschichten, was aufgrund der genannten Antihafteigenschaft eine Minderung der Thrombosengefahr zur Folge hat. Des weitern eignet sich die erfindungsgemässe Legierung auch für die Beschichtung derjenigen Teile von medizinischen und analytischen Geräten, welche bei der Benutzung der Geräte in Kontakt mit Proteinen, proteinhaltigen Lösungen und/oder Körperflüssigkeiten kommen. Die Vorteile dabei sind, dass Messungenauigkeiten aufgrund von anhaftenden proteinhaltigen Verunreinigungen vermieden werden und die regelmässige Wartung und Reinigung der Geräte vereinfacht wird. Die erfindungsgemässe Beschichtung kann auch auf die Haut oder Schleimhaut durchstossende Implantate aufgebracht werden und zwar an der Stelle, an der diese zum tierischen oder menschlichen Körper austreten. Dadurch wird die Gefahr einer Infektion entlang des Implantates reduziert.

Die erfindungsgemässen Legierungen können auch als Korrosions- und/oder Wärmeschutzschicht für Maschinen- und Anlageteile eingesetzt werden, so zum Beispiel im Turbinenbau als Korrosionsschutz- und Wärmedämmschichten für thermisch mässig, d.h. bis ca. 610° C, beanspruchte Schaufeln. Die Schaufeln sind dabei vorzugsweise aus Titanwerkstoffen und es gelangt vorzugsweise das Vakuumplasmaspritz-Verfahren zum Einsatz. Für andere chemisch und thermisch beanspruchte Teile in Energiegewinnungsanlagen und Rauchgasreinigungsanlagen werden die erfindungsgemässen Legierungen mittels thermischen Spritzverfahren oder mittels PVD-Technik abgeschieden. Die erfindungsgemässe Legierung kann dabei Bestandteil eines ganzen Schichtsystems sein.

## Patentansprüche

1. Antihaftbeschichtung für insbesondere metallische Festkörper, **dadurch gekennzeichnet, dass** sie aus einer titanhaltigen Legierung mit einer quasikristallinen Phase besteht, deren Anteil mindestens 50 Vol.% beträgt.

2. Beschichtung nach Anspruch 1, **dadurch gekennzeichnet, dass** sie Legierungsbestandteile mit folgenden Atomprozenten aufweist:
| | |
|---|---|
| 35 - 65 at.% | Ti |
| 8 - 50 at.% | Zr |
| 10 - 30 at.% | eines oder mehrerer der Elemente Ni, Co, Fe, Mn, Pd, Ag |

3. Beschichtung nach Anspruch 2, **gekennzeichnet durch** mindestens eine der Legierungen Ti₆₅Fe₂₅Zr₁₀; Ti₆₀Fe₂₅Zr₁₅; Ti₅₃Co₂₀Zr₂₇; Ti₆₅Ni₂₅Zr₁₀; Ti₆₀Ni₂₅Zr₁₅; Ti₅₃Ni₂₀Zr₂₇; Ti₅₀Ni₁₉Zr₃₁; Ti₄₅Ni₁₇Zr₃₈ Ti₄₄Ni₁₆Zr₄₀ und Ti_{41.5}Ni₁₇Zr_{41.5}

4. Beschichtung nach Anspruch 1, **dadurch gekennzeichnet, dass** sie Legierungsbestandteile mit folgenden Atomprozenten aufweist:
| | |
|---|---|
| 40 - 75 at.% | Ti |
| 3 - 25 at.% | Si |
| 15 - 50 at.% | eines oder mehrerer der Elemente Cr, Mn, V, Fe, Nb, Mo, Ta |
| 0 - 17 Gew.% | O₂ zur Stabilisierung der quasikristallinen Struktur. |

5. Beschichtung nach Anspruch 2, **gekennzeichnet durch** mindestens eine der Legierungen Ti₆₈Fe₂₆Si₆, Ti₆₀Mn₃₇Si₃ und Ti₆₄Cr₃₂Si₄; oder
Ti₆₈₋ₓCr₃₂Siₓ, wobei für x gilt 6 < x < 18;
Ti₇₃₋ₓV₂₇Siₓ, wobei für x gilt 13 < x < 25;
Ti₇₅₋ₓCr₂₅Siₓ, wobei für x gilt 10 < x < 20.

6. Beschichtung nach einem der Ansprüche 1 bis 5, **dadurch gekennzeichnet, dass** sie eine Dicke von 0.1 µm bis 500 µm aufweist.

7. Verwendung einer quasikristallinen Beschichtung gemäss einem der Ansprüche 1 bis 6 zur Erniedrigung der Hafteigenschaft eines Substrates für Proteine und Gewebebestandteile oder zum Schutz eines Substrates vor proteinhaltigen Lösungen und Gewebeflüssigkeiten.

8. Für die operative Knochenbehandlung dienendes Implantat, **dadurch gekennzeichnet, dass** es mit einer Beschichtung gemäss einem der Ansprüche 1 bis 6 überzogen ist.

9. Gefässimplantat für tierische und menschliche Gewebe und Organe, **dadurch gekennzeichnet, dass** es mit einer Beschichtung gemäss einem der Ansprüche 1 bis 6 überzogen ist.

10. Zum Einsetzen in einen menschlichen oder tierischen Körper dienende Prothese, **dadurch gekennzeichnet, dass** es mindestens partiell mit einer Beschichtung gemäss einem der Ansprüche 1 bis 6 überzogen ist.

11. Zum Einsetzen in einen menschlichen oder tierischen Körper dienendes, die Haut durchstossendes Implantat, **dadurch gekennzeichnet, dass** es mindestens partiell mit einer Beschichtung gemäss einem der Ansprüche 1 bis 6 überzogen ist.

12. Zum Einsetzen in einen menschlichen oder tierischen Körper dienendes, die Schleimhaut durchstossendes Implantat, **dadurch gekennzeichnet, dass** es mindestens partiell mit einer Beschichtung gemäss einem der Ansprüche 1 bis 6 überzogen ist.

13. Verwendung einer quasikristallinen Beschichtung gemäss einem der Ansprüche 1 bis 6 als Korrosionsschutzschicht für thermisch bis max. 610°C beanspruchte Maschinenund Anlageteile zur Energiegewinnung und Rauchgaswäsche.

14. Verwendung einer quasikristallinen Beschichtung gemäss einem der Ansprüche 1 bis 6 als Wärmeschutzschicht für thermisch bis max. 610°C beanspruchte Maschinen- und Anlageteile zur Energiegewinnung und Rauchgaswäsche.

15. Verwendung einer quasikristallinen Beschichtung gemäss einem der Ansprüche 1 bis 6 als Korrosionsschutzschicht für thermisch bis max. 610°C beanspruchte Turbinenschaufeln.

16. Verwendung einer quasikristallinen Beschichtung gemäss einem der Ansprüche 1 bis 6 als Wärmeschutzschicht für thermisch bis max. 610°C beanspruchte Turbinenschaufeln.

17. Eine quasikristalline Beschichtung gemäss den Ansprüchen 13 bis 16 als Bestandteil eines Schichtsystems.
